# EUROPÄISCHE PATENTSCHRIFT

(11) **EP 3 019 589 B1**
(45) Veröffentlichungstag und Bekanntmachung des Hinweises auf die Patenterteilung: **17.05.2017**
(21) Anmeldenummer: 14739097.5
(22) Anmeldetag: 03.07.2014
(51) Int. Cl.: C12M 3/06, C12M 1/00, C12M 1/12, C12M 1/34

(54) **ZIRKULATIONSSYSTEM SOWIE VERFAHREN ZUR VITALVERSORGUNG VON ZELLKULTUREN IN EINEM MIKROFLUIDISCHEN NETZWERK**
CIRCULATION SYSTEM AND METHOD FOR VITAL SUPPLY OF CELL CULTURES IN A MICROFLUIDIC NETWORK
SYSTÈME DE CIRCULATION AINSI QUE PROCÉDÉ D'ALIMENTATION VITALE DE CULTURES CELLULAIRES DANS UN RÉSEAU MICROFLUIDIQUE

(30) Priorität: 10.07.2013 DE 102013011768
(43) Veröffentlichungstag der Anmeldung: 18.05.2016
(73) Patentinhaber: Fraunhofer Gesellschaft zur Förderung der angewandten Forschung E.V., 80686 München (DE)
(72) Erfinder: BUSEK, Mathias, 01277 Dresden (DE); SONNTAG, Frank, 01277 Dresden (DE); KLOTZBACH, Udo, 01326 Dresden (DE)
(74) Vertreter: Pfenning, Meinig & Partner mbB
(86) Internationale Anmeldenummer: PCT/EP2014/064192
(87) Internationale Veröffentlichungsnummer: WO 2015/003997

(56) Entgegenhaltungen:
- WO-A1-2012/016711
- WO-A2-03/027223
- WO-A2-03/085379
- WO-A2-2007/047772
- WO-A2-2008/025351
- DE-A1-102006 030 068
- US-A1- 2006 199 260

## Beschreibung

Die vorliegende Erfindung betrifft den Bereich mikrofluidischer Zellkultursysteme, insbesondere ein Zirkulationssystem sowie ein Verfahren zur Vitalversorgung solcher Zellkulturen.

Zum Prüfen und Testen von Arzneimitteln, Chemikalien oder Kosmetika, sowie bei der Suche nach Wirkstoffen oder deren Wirkung hervorrufende Signalwege werden klassische Tierversuche mehr und mehr durch Bioassays verdrängt. Nicht zuletzt aus ethischen als auch ökonomischen Gründen werden daher Zellkultursysteme gefordert, mit denen aussagekräftige Daten gewonnen werden können, die vergleichbar sowie reproduzierbar sind. Die Bestrebungen führen daher zu Zellkultursystemen, mit denen artgleiche Zelltypen bzw. unterschiedliche Zelltypen eines Individuums oder Organismus in geschlossenen Kreisläufen kultiviert werden können. Zur Nachahmung metabolischer Prozesse werden dabei verschiedene Zelltypen, wie beispielsweise Cardiozyten, Hepatozyten, Osteozyten oder Zellen weiterer, bspw. neuronaler (afferente oder efferente) Gewebe, in Kompartimenten räumlich voneinander getrennt, in mikrofluidischen Netzwerken mit Nährstoffen versorgt und begast. Vorwiegend gestaltet sich die Kultivierung solcher komplexen Bioassays jedoch dadurch problematisch, dass für die einzelnen verschiedenen Zelltypen unterschiedliche Kulturbedingungen erforderlich sind.

EP 2 505 638 A1 offenbart ein aus neuronalen und somatischen Zellen gebildetes Bioassay. Zur Ausbildung eines neuronalen Affektor-Effektor-Netzwerks sind die verschiedenen Zelltypen dabei in einem fluidischen Kreislauf nacheinander, d.h. in Reihe in entsprechenden Kavitäten angeordnet. Zur Inkubation der verschiedenen Zelltypen werden die Kavitäten vorzugsweise vom gemeinsamen fluidischen Kreislauf getrennt. Infolge der Reihenanordnung der einzelnen Kavitäten bzw. Kompartimente wird dabei auch der Fluidkreislauf der gemeinsamen Vitalversorgung vollständig unterbrochen, so dass die einzelnen Zellkulturen außerhalb des Netzwerks aufwendig separat stimuliert und versorgt werden müssen. Nachteilig ist weiterhin, dass die jeweiligen Zellen enthaltenden Kavitäten innerhalb der fluidischen Reihenanordnung einem gemeinsamen Volumenstrom und somit einem gemeinsamen hydraulischen Druck ausgesetzt sind. Dies kann sich jedoch negativ auf die unterschiedlichen Bedürfnisse der verschiedenen Zelltypen auswirken und somit die Adhärenz, Differenzierung und Proliferation der zuvor aufwendig inkubierten Zelltypen negativ beeinflussen. Um optimale Bedingungen zur Ausdifferenzierung und Proliferation spezieller Zelltypen insbesondere in geschlossenen Kreisläufen bereitzustellen ist es daher erforderlich, für möglichst jedes Kompartiment differenzierte Kulturbedingungen mit möglichst großer nähe zu natürlichen (physiologischen) Bedingungen zu gewährleisten.

Aus WO 2012/016711 A1 ist ein geschlossenes Zirkulationssystem sich aufzweigender Kapillaren zur Bildung und Versorgung biologischer Gewebsstrukturen bekannt. Mittels einer Pumpe wird dabei ein physiologisches Fluid pulsierend durch einen arteriell strukturierten bzw. sich aufzweigenden Teil eines Kreislaufs gepumpt, der in einem Zellkulturen enthaltenden Kompartiment mündet, wobei venöses Fluid aus dem Zellkulturkompartiment in einen venös strukturierten Teil mündet. Der arterielle- und der venöse Teil bilden dabei einen geschlossen Kreislauf. Schwankungen des Drucks und des Volumenstroms innerhalb dieses Kreislaufs sollen dadurch verhindert werden, dass der Volumenstrom an einem arteriellen Verzweigungspunkt jeweils zu gleichen Teilen aufgeteilt in einem venösen Mündungspunkt zu gleichen Teilen wieder zusammengeführt wird. Durch entsprechend dimensionierte Querschnitte von Mikrokanälen wird eine definierte Strömungsaufteilung erreicht. Es ist also davon auszugehen, dass der effektive Volumenstrom im Kreislauf konstant ist, so dass auch die parallel angeordneten Zellkulturkompartimente mit einem konstanten Volumenstrom versorgt werden.

Für solche Zellkultursysteme, bei denen mehrere Zellkulturkompartimente mit unterschiedlichen Zellkulturen aus einem zentralen geschlossenen Fluidkreislauf versorgt werden ist es jedoch erforderlich, die Volumenströme und Drücke innerhalb des Kreislaufs an die jeweiligen Bedürfnisse der verschiedenen Zelltypen anzupassen, um eine optimale Adhärenz, Differenzierung und/oder Proliferation von allen in diesem geschlossenen System vorhandenen Zellkulturnen zu erreichen. Neben hydraulischen Stimuli wie statische und dynamische Druckbeaufschlagung sollten auch weitere physiologische Parameter, wie beispielsweise pH-Wert, Gassättigung (Sauerstoffpartialdruck, Kohlendioxidpartialdruck) oder Glukose-Konzentration möglichst vor unmittelbarem Kontakt mit den Zellkulturen geregelt werden können.

So sind aus WO 03/085379 A2 ein mikrofluidisches Partikel-Analysesystem, aus DE 10 2006 030 068 A1 eine Vorrichtung und ein Verfahren zur Zu- und Abfuhr von Fluiden in geschüttelten Mikrireaktoren Arrays und aus US 2006/0199260 A1 ein mikrobiologischer Reaktor für eine kontinuierliche Zellkultivierung bekannt.

WO 2007/047772 A2 betrifft eine mikrofluidische Zellkultivierungsvorrichtung.

Ein Verfahren zur Herstellung eines Biorektors oder Lab-on-Chip-Systems sowie damit hergestellte Bioreaktoren oder Lab-on-Chip-Systemen sind in WO 2008/025351 A2 und eine Vorrichtung und ein Verfahren für pharmakinetische Basiszellkultursysteme in WO 03/027223 A2 beschrieben.

Die Offenbarung von WO 2012/016711 A1 betrifft ein Zirkulationssystem.

Es ist daher Aufgabe der vorliegenden Erfindung, eine Vorrichtung und ein Verfahren zur Vitalversorgung von Zellkulturen in einem Zellkultursystem bereitzustellen, wobei eine separate definierte Versorgung einzelner Zellkulturen möglich ist.

Die Aufgabe wird mit einem Zirkulationssystem nach Anspruch 1 und aus verfahrenstechnischer Sicht nach Anspruch 12 gelöst. Vorteilhafte Ausgestaltungen der vorliegenden Erfindung können mit Merkmalen der untergeordneten Ansprüche erreicht werden.

Erfindungsgemäß wird zur Vitalversorgung von Zellkulturen in einem mikrofluidischen Netzwerk ein Zirkulationssystem vorgeschlagen, das mehrere Zellkultur-Kompartimente enthält, die jeweils mindestens einen Eingang und mindestens einen Ausgang aufweisen. Weiterhin weist das Zirkulationssystem mindestens ein Nährlösung enthaltendes Reservoir auf, das über eine erste Mikrokanalstruktur, die zumindest eine Verzweigung erster Ordnung aufweist, mit mindestens einem Eingang jedes Zellkultur-Kompartiments verbunden ist. Außerdem ist mindestens eine Fördereinrichtung enthalten, mit der die Nährlösung aus dem Reservoir über die Mikrokanalstruktur in die Zellkultur Kompartimente förderbar ist. Erfindungsgemäß ist das Zirkulationssystem dadurch gekennzeichnet, dass in jedem Arm der Verzeigung erster Ordnung und/oder im Verlauf jedes unmittelbar in einen Eingang eines Zellkultur-Kompartiments mündenden Mikrokanals der ersten Mikrokanalstruktur eine Einrichtung zur Volumenstromregelung angeordnet ist, mit der ein Volumenstrom der Nährlösung regelbar ist.

Gegenüber dem Stand der Technik liegt der Kern der vorliegenden Erfindung daher in der Möglichkeit, dass der Volumenstrom einer Nährlösung, die aus einem zentralen Reservoir durch ein sich verzweigendes mikrofluidischen Netzwerk förderbar ist bzw. gefördert wird, unmittelbar vor dem Einlass in jedes Zellen enthaltende Kompartiment regelbar ist. Vorteilhafterweise kann der Volumenstrom der Nährlösung und weiterer Fluide dabei in Abhängigkeit des Nährstoffbedarfs jedes einzelnen Kompartiments geregelt werden. Als Kompartimente sollen vorzugsweise zylindrische oder sphärische Räume verstanden werden, in denen Bedingungen und Möglichkeiten zur Adhärenz von Zellen gegeben sind. Ein Kompartiment kann daher eine entsprechende Oberflächenbeschaffenheit bzw. eine Beschichtung aufweisen. In einem Kompartiment können aber auch Kollagen-Strukturen (scaffolds) enthalten sein, die eine dreidimensionale Proliferation von Zellen ermöglichen. Es kann sich bei den Kompartimenten allerdings auch um Kavitäten handeln, in denen flüssige Zellkulturen enthalten sind.

Vorzugsweise sind die Zellkultur-Kompartimente in dem Zirkulationssystem (analog elektrotechnischer Anordnungen) parallel zueinander angeordnet, wobei im Anschluss an eine Verzweigung erster Ordnung, d.h. an einem Arm der Verzweigung bzw. im Verlauf der sich verzweigenden ersten Mikrokanalstruktur, auch mehrere der Zellkultur-Kompartimente in Reihe angeordnet sein können und/oder zumindest durch eine Mikrokanalverbindung zwischen mindestens einem Ausgang eines ersten Zellkultur-Kompartiments mit mindestens einem Eingang eines weiteren Zellkultur-Kompartiments verbunden sein. Dabei kann die Einrichtung zur Volumenstromregelung auch im Verlauf einer Mikrokanalverbindung der ersten Mikrokanalstruktur angeordnet sein, die mindestens zwei Zellkultur-Kompartimente in Reihe miteinander verbindet.

Wie bereits beschrieben, weist die erste Mikrokanalstruktur mindestens eine Verzweigung erster Ordnung auf, wobei darunter zu verstehen ist, dass sich die Mikrokanalstruktur zumindest einmal in mindestens zwei Mikrokanäle verzweigt. Dabei kann die Verzweigung erster Ordnung eine Mehrzahl von Armen aufweisen, die vorzugsweise jeweils unmittelbar in einen Eingang eines Zellkultur-Kompartiments münden.

In einer vorteilhaften Ausführungsform kann die erste Mikrokanalstruktur im Verlauf mindestens eines Arms der Verzweigung erster Ordnung mindestens eine weitere Verzweigung zweiter Ordnung aufweisen, die ebenfalls mehrere Arme aufweisen kann, von denen jeder Arm mit mindestens einem Eingang eines Zellkultur-Kompartiments verbunden ist.

Bei einer weiteren vorteilhaften Ausführungsform des erfindungsgemäßen Zirkulationssystems kann mindestens ein Arm einer Verzweigung zweiter Ordnung mindestens eine weitere Verzweigung dritter Ordnung aufweisen, deren Arme jeweils in mindestens einen Eingang eines Zellkulturkompartiments münden.

Denkbar ist weiterhin, dass mehrere Arme einer Verzweigung erster, zweiter und/oder dritter Ordnung in mehrere Eingänge eines Zellkultur-Kompartiments münden, wobei im Verlauf aller unmittelbar in ein Zellkultur-Kompartiment mündenden Arme, d.h. Mikrokanälen der ersten Mikrokanalstruktur - durch die die Nährlösung in das Zellkultur-Kompartiment gefördert wird - eine Einrichtung zur Volumenstromregelung vorhanden ist.

Das gesamte Zirkulationssystem kann im Mehrschichtaufbau realisiert sein, wobei mehrere Schichten miteinander laminierbar und flächig stoffschlüssig mit einander verbunden sind und in mindestens einer Schicht entsprechende Aussparungen für Mikrokanalstrukturen, für mindestens ein Reservoir, mindestens eine Fördereinrichtung, Zellkultur-Kompartimente und Einrichtungen zur Volumenstromregelung ausgebildet sind. Die Aussparungen der übereinander angeordneten Schichten bilden dabei Zwischenräume der genannten Strukturen aus, die in einem Netzwerk miteinander korrespondieren. Somit können mehrere dreidimensionale Mikrokanalstrukturen auf engstem Raum mit entsprechenden Zellkultur-Kompartimenten realisiert sein. Die einzelnen Schichten können beispielsweise aus Polydimethylsiloxan (PDMS), Polycarbonant (PC), Polypropylen (PP) oder Polyethylenterephthalat (PET) gebildet sein.

Unter Mikrokanälen sollen solche Kanäle verstanden werden, deren Durchmesser 1 mm oder durchströmbare Querschnittsfläche von 1 mm² nicht übersteigt.

Mikrokanäle erster, zweiter und dritter Ordnung sollten eine maximale Länge von 1 mm bis 100 mm aufweisen.

Die Volumina der Zellkulturkompartimente können im Bereich von 1 mm³ bis 100 mm³ variieren.

Die Einrichtung(en) zur Volumenstromregelung kann/können mit einer zweiten Mikrokanalstruktur verbunden sein, über die mindestens ein Fluid und/oder mindestens eine Substanz in einen Mikrokanal der ersten Mikrokanalstruktur in dosierter Form zuführbar ist/sind. Hierzu kann/können die Einrichtung(en) zur Volumenstromregelung mit einem MEMS(micro-electromechanical-system)-basierten Mikroventil realisiert sein, das die dosierte Abgabe aus der zweiten Mikrokanalstruktur in die erste Mikrokanalstruktur gewährleistet.

Entsprechend kann/können die Einrichtung(en) zur Volumenstromregelung auch mit einem magentischen, elektromagnetischen, elektromotorischen pneumatischen, elektropneumatischen, hydraulischen oder elektrohydraulischen Ventil (Mikroventil) realisiert sein. Die Einrichtung(en) zur Volumenstromregelung ist/sind_mit einem Membranventil realisiert, das eine flexiblen Membran aufweist. Dadurch kann das Ventil mit einem von außen aufgebrachten pneumatischen oder hydraulischen Druck betätigt werden.

Vorteilhafterweise kann die flexible Membran als Trennwand zwischen der ersten Mikrokanalstruktur und der zweiten Mikrokanalstruktur ausgebildet sein, wobei die flexible Membran über die zweite Mikrokanalstruktur mit einem aktivierbaren/deaktivierbaren vorgebbaren pneumatischen und/oder vorgebbaren hydraulischen Druck beaufschlagbar ist. Bei einer druckabhängigen (pneumatischen oder hydraulischen) Wölbung/Dehnung der flexiblen Membran kann eine Änderung der von der Nährlösung durchströmbaren Querschnittsfläche eines Mikrokanals der ersten Mikrokanalstruktur erreicht werden. Mit entsprechenden Mitteln kann ein Mikrokanal der zweiten Mikrokanalstruktur daher so mit Überdruck beaufschlagt werden, dass eine in den Mikrokanal der ersten Mikrokanalstruktur hineinragende definierte Wölbung der flexiblen Membran erreicht werden kann, die eine definierte Querschnittsverengung des entsprechenden Mikrokanals der ersten Mikrokanalstruktur hervorruft. Infolge der Variation der von der Nährlösung durchströmbaren Querschnittsfläche, kann der Volumenstrom der Nährlösung in dem entsprechenden Mikrokanal eingestellt werden.

Es ist anzumerken, dass jede Einrichtung zur Volumenstromregelung unabhängig von weiteren in dem Netzwerk vorhandenen Einrichtungen zur Volumenstromregelung betrieben werden kann. Eine zweite Mikrokanalstruktur ist daher jeweils nur mit einer einzigen Einrichtung zur Volumenstromregelung verbunden, so dass die Anzahl der zweiten Mikrokanalstrukturen mit der Anzahl der Einrichtungen zur Volumenstromregelung übereinstimmen. Bei einer vorteilhaften Ausführungsform können jedoch auch sich verzweigende zweite Mikrokanalstrukturen eingesetzt werden, wobei eine zweite Mikrokanalstruktur mit mehreren Einrichtungen zur Volumenstromregelung verbunden sind. In diesem Fall können dann Gruppen von Einrichtungen zur Volumenstromregelung unabhängig voneinander betrieben werden.

Eine definierte Wölbung der flexiblen Membran kann auch durch einen an der zweiten Mikrokanalstruktur aufgebrachten Unterdruck erreicht werden, wobei die Wölbung in den Mikroanal der zweiten Mikrokanalstruktur hineinragt. In diesem Fall kann der erste Mikrokanal, durch den die Nährlösung gefördert wird, so ausgestaltet sein, dass der Durchfluss bei Normaldruck (es liegt kein Über- bzw. Unterdruck an der zweiten Mikrokanalstruktur an) durch die flexible Membran gesperrt ist. Erst bei Anliegen eines Unterdrucks an der zweiten Mikrokanalstruktur wird die flexible Membran so definiert gewölbt, dass in dem ersten Mikrokanal eine definierte durchströmbare Querschnittsfläche erreicht wird, durch die der Volumenstrom der Nährlösung verändert wird.

Es ist weiterhin besonders vorteilhaft, wenn die flexible Membran aus einem selektivpermeablen Material gebildet ist, das für mindestens ein Fluid, vorzugsweise ausgewählt aus der Gruppe aus Stickstoff, Sauerstoff, Kohlendioxid und/oder mindestens eine Substanz, vorzugsweise ausgewählt aus der Gruppe aus Stickstoff, Phosphat, Glucose, HSA (numanes Serumalbumin), Wachstumsfaktoren, wie EGF (endothelial growth factor), VEGF (vascular endothelial growth factor) oder NGF (nerve growth factor) permeabel ist, so dass ein Austausch des mindestens einen Fluids und/oder der mindestens einen Substanz zwischen einem Kanal der ersten Mikrokanalstruktur und der zweiten Mikrokanalanalstruktur realisierbar ist. Als Fluid werden dabei Flüssigkeiten als auch Gase verstanden. Die selektivpermeable Membran kann aber auch als Port zum Austausch von niedermolekularen Testsubstanzen, wie beispielsweise Medikamenten dienen, deren Einfluss auf ein jeweiliges Zellkultur-Kompartiment bzw. den darin kultivierten Zellen getestet werden soll.

Durch die selektivpermeable Eigenschaft der flexiblen Membran können die Einrichtungen zur Volumenstromregelung zusätzlich auch als individuelle Dosierer für die einzelnen Zellkultur-Kompartimente eingesetzt werden, wobei individuelle Bedürfnisse der verschiedenen Zellkulturen berücksichtigt werden können. Dabei ist ein Substanz- und/oder Fluid-Austausch in beide Richtungen oder nur in eine Richtung durch die Membran möglich. So kann beispielsweise eine Erhöhung des Sauerstoffpartialdrucks einer in dem ersten Mikrokanal strömenden Nährlösung dadurch erreicht werden, dass die zweite Mikrokanalstruktur mit einem erhöhten Sauerstoffpartialdruck beaufschlagt wird, wobei Sauerstoff durch die für Sauerstoff permeable flexible Membran in den ersten Mikrokanal und somit in die Nährlösung gelangt. Gleichzeitig kann im ersten Mikrokanal der Volumenstrom der Nährlösung durch die Wölbung der flexiblen Membran verändert werden. Es kann auch eine Senkung des Sauerstoffpartialdrucks erreicht werden, in dem der zweite Mikrokanal mit geringem Sauerstoffpartialdruck beaufschlagt wird, wobei der Nährlösung beim Vorbeiströmen an der flexiblen Membran Sauerstoff entzogen wird. Dabei sollte am zweiten Mikrokanal möglichst Unterdruck anliegen. Die Einrichtungen zur Volumenstromregelung können den Einsatz von Oxygenatoren/Deoxygenatoren somit vollständig ersetzten.

Die selektivpermeable flexible Membran kann aus einem Kunststoffmaterial, wie beispielsweise Polydimethylsiloxan (PDMS), Polycarbonant (PC), Polypropylen (PP) oder Polyethylenterephthalat (PET) gebildet sein und auf einer der Nährlösung zugewandten Seite, eine amphiphile Oberflächenbeschichtung aufweisen, so dass eine möglichst geringe Adhäsion von in der Nährlösung enthaltenden Substanzen und/oder ausgeschwemmten Zellen erreicht werden kann.

Der Volumenstrom bzw. die Durchströmung durch die einzelnen Kanäle der Mikrokanalstruktur kann entweder direkt durch berührungslose Strömungsmesstechnik, wie z.B. Particle Image Velocimetry (PIV) oder via Laser-Doppler Anemometrie (LDA) erfasst werden. Der Druck im Mikrokanal kann durch Wölbung an einer dafür vorgesehenen Messmembran oder an der flexiblen selektivpermeablen Membran erfasst werden. Weiterhin können die Einrichtungen zur Volumenstromregelung sensorische Mittel aufweisen, mit denen die Wölbung/Dehnung der flexiblen Membran sowie Drücke, Partialdrücke und/oder Volumenströme der Nährlösung vorzugsweise berührungslos erfasst werden können. Die Wölbung kann beispielsweise optisch, mit konfokalen Sensoren und/oder einem Laser-Interferometer erfasst werden, die in den Einrichtungen zur Volumenstromregelung vorgesehen sind. Mit weiteren optischen Messverfahren, wie z.B. Verfahren zur Bestimmung der Fluoreszenzlebensdauer oder SPR (surface plasmon resonance) können auch Fluide und/oder Konzentrationen von Fluiden und/oder Substanzen erfasst werden, die in der Nährlösung enthalten sind und/oder in dosierter Form zugeführt werden. Anhand der mit den genannten Sensoren erfassten Messwerte können die pneumatischen und/oder hydraulischen Drücke, die zur Wölbung der flexiblen Membran auf die zweite Mikrokanalstruktur beaufschlagt werden, so eingestellt werden, dass eine definierte Volumenströmung der Nährlösung erreicht werden kann. Neben den berührungslosen Sensoren können auch elektrochemische Sensoren oder Sensoren eingesetzt werden, die auf leitfähigen Materialien beruhen.

Auch das Dosieren von Fluiden und/oder Substanzen kann anhand der mit den Sensoren erfassten Messwerte geregelt werden. Hierzu kann die zweite Mikrokanalstruktur jeweils mit einem entsprechenden Fluid(Gas)-Mischer oder Substanzmischer verbunden sein.

Entsprechend eines geschlossenen Kreislaufs des erfindungsgemäßen Zirkulationssystems, weist jedes in dem Zirkulationssystem vorhandene Zellkultur-Kompartiment mindestens einen Ausgang auf, der über eine Mikrokanalverbindung mit mindestens einem Mündungspunkt einer dritten Mikrokanalstruktur verbunden ist, die in das Nährlösung enthaltende Reservoir mündet.

Vorzugsweise sind die erste Mikrokanalstruktur und die dritte Mikrokanalstruktur spiegelbildlich ausgebildet

Selbstverständlich können die Einrichtungen zur Volumenstromregelung auch im Verlauf jeder Mikrokanalverbindungen angeordnet sein, die einen Ausgang eines Zellkultur-Kompartiments mit einem Mündungspunkt der dritten Mikrokanalstruktur verbindet.

Bei einer vorteilhaften Ausführungsform des erfindungsgemäßen Zirkulationssystems kann im Verlauf der ersten Mikrokanalstruktur vor der mindestens einen Verzweigung erster Ordnung und/oder in jedem Arm der Verzeigung erster Ordnung und/oder im Verlauf jedes unmittelbar in einen Eingang der Zellkultur-Kompartimente mündenden Mikrokanals der ersten Mikrokanalstruktur, mindestens ein erster Oxygenator/Deoxygenator und/oder im Verlauf jeder aus einem Ausgang eines Zellkultur-Kompartiments in eine dritte Mikrokanalstruktur führende Mikrokanalverbindung vor einem Mündungspunkt und/oder dahinter im Verlauf der dritten Mikrokanalstruktur ein zweiter Oxygenator/Deoxygenator angeordnet sein. Auf diese Weise können im Verlauf der ersten Mikrokanalstruktur über ein Zellkultur-Kompartiment bis zur dritten Mikrokanalstruktur, unabhängig vom Volumenstrom der Nährlösung, definierte Gradienten von Sauerstoff-, Kohlendioxid und/oder Stickstoffpartialdrücken erreicht werden.

Zur Bereitstellung des Förderstroms der Nährlösung kann als Fördereinrichtung eine Peristaltikpumpe eingesetzt werden, die vorzugsweise vor einer Verzweigung erster Ordnung im Verlauf der ersten Mikrokanalstruktur angeordnet ist.

Bei dem erfindungsgemäßen Verfahren zur Vitalversorgung von Zellkulturen in einem mikrofluidischen Netzwerk, wird eine Nährlösung mit einer Fördereinrichtung aus einem Reservoir über mindestens eine Verzweigung erster Ordnung einer sich verzweigenden Mikrokanalstruktur eingangsseitig in mehrere Zellkultur-Kompartimente gefördert, wobei der Volumenstrom der Nährlösung in jedem Arm der Verzeigung erster Ordnung und/oder im Verlauf jedes unmittelbar in einen Eingang eines Zellkultur-Kompartiments mündenden Mikrokanals der Mikrokanalstruktur separat geregelt wird.

Bei dem erfindungsgemäßen Verfahren wird eine Einrichtung zur Volumenstromregelung eingesetzt, die ein Membranventil mit einer flexiblen Membran enthält, wobei die flexible Membran als Trennwand zwischen der ersten Mikrokanalstruktur und einer zweiten Mikrokanalstruktur ausgebildet ist. Wird über die zweite Mikrokanalstruktur ein aktivierbarer/deaktivierbarer vorgebbarer pneumatischer und/oder vorgebbarer hydraulischer Druck beaufschlagt, kann die flexible Membran derart gewölbt/gedehnt werden, dass die von der Nährlösung durchströmbare Querschnittsfläche des Mikrokanals der ersten Mikrokanalstruktur verändert wird.

Vorteilhafterweise kann als flexible Membran eine aus PDMS, Polycarbonant (PC), Polypropylen (PP) oder Polyethylenterephthalat (PET) gebildete selektivpermeable Membran verwendet werden, über die zwischen einem Mikrokanal der ersten Mikrokanalstruktur und der zweiten Mikrokanalanalstruktur bzw. einem Mikrokanal der zweiten Mikrokanalstruktur mindestens ein Fluid, vorzugsweise ausgewählt aus der Gruppe aus Stickstoff, Sauerstoff, Kohlendioxid und/oder mindestens eine Substanz, vorzugsweise ausgewählt aus der Gruppe aus Phosphat, Glucose, HSA oder EGF ausgetauscht und/oder dosiert wird. Selbstverständlich kann die selektivpermeable Membran auch so ausgebildet sein, dass sie zum Austausch nur einer Testsubstanz, wie z.B. niedermolekularen Strukturen geeignet ist. Bei solchen niedermolekularen Strukturen kann es sich vorzugsweise um Wirkstoffe oder Medikamente handeln, deren Einfluss auf Zellkultur-Kompartimente bzw. auf die darin kultivierten Zellen getestet werden sollen.

Zur Regelung der Volumenströmung der Nährlösung werden die Wölbung/Dehnung der flexiblen Membran sowie Drücke, Partialdrücke und/oder Volumenströme der Nährlösung mit berührungslosen optischen Sensoren erfasst. Die Wölbung wird mit konfokalen Sensoren und/oder einem Laser-Interferometer erfasst, wobei der Volumenstrom bzw. die Durchströmung auch direkt mit PIV oder LDA detektiert werden kann. Mit weiteren Sensoren oder Messverfahren, wie z.B. Verfahren und Sensoren zur Erfassung von Fluoreszenz bzw. Fluoreszenzlöschung sowie SPR-Methoden können auch in der Nährlösung enthaltene oder zugeführte Fluide und/oder Konzentrationen von Fluiden und/oder Substanzen erfasst werden.

Die Volumenstromregelung in den Mikrokanälen wird also so realisiert, dass zunächst ein Volumenstrom-Ist-Wert der geförderten Nährlösung mit Hilfe einer der optischen Sensoren der Einrichtung zur Volumenflussregelung erfasst wird. Anschließend wird die zweite Mikrokanalstruktur bzw. ein Mikrokanal der Mikrokanalstruktur mit einem definierten pneumatischen bzw. hydraulischen Druck beaufschlagt, bis eine Wölbung der flexiblen Membran erreicht ist, die eine Verengung des von der Nährlösung durchströmbaren Querschnitts verursacht und den Volumenstrom der Nährlösung dadurch begrenzt.

Weiterhin können Ist-Werte zu regelnder bzw. zu dosierender Substanzen oder Vitalparameter (Sauerstoffsättigung, LDH (Lactatdehydrogenase), NADH (Nicotinamidadenindinukleotid)) erfasst werden und anschließend durch die beschriebene Volumenstromregelung auf Basis eines Massentransportmodels auf einen vorgebbaren Soll-Wert eingestellt werden. Vorteilhafterweise kann dadurch eine bedarfsgerechte Nährstoffzugabe und eine gezielte Beaufschlagung mit Testsubstanzen erreicht werden.

Nachfolgend wird die vorliegende Erfindung anhand von Ausführungsbeispielen und Figuren näher erläutert. Es zeigen:
Figur 1: eine schematische Darstellung eines Ausführungsbeispiels des erfindungsgemäßen Zirkulationssystems
Figur 2a/b: schematische Schnittdarstellungen einer Einrichtung zur Volumenstromregelung im Über- und Unterdruckbetrieb
Figur 3a/b: schematische Schnittdarstellungen der Einrichtung zur Volumenstromregelung in gegossener Ausführung a) und als Mehrschicht-Ausführung b)

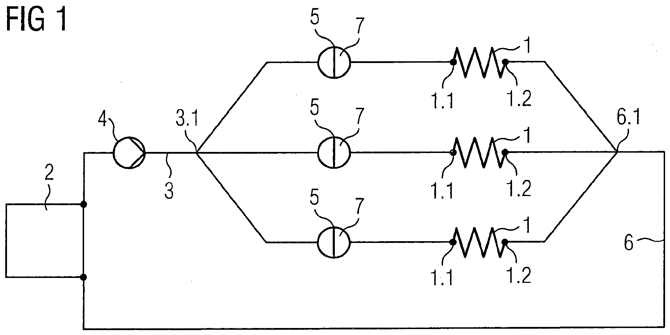

Die Figur 1 zeigt eine schematische Darstellung des erfindungsgemäßen Zirkulationssystems, bei dem ein Reservoir 2, in dem eine Nährlösung enthalten ist, über eine sich aufzweigende Mikrokanalstruktur 3 mit jeweils mindestens einem Eingang 1.1 mehrerer Zellkultur-Kompartimente 1 verbunden ist. Im Verlauf der ersten Mikrokanalstruktur 3 ist eine Fördereinrichtung 4 in Form einer Peristaltikpumpe zur Förderung der Nährlösung angeordnet. In jedem Arm bzw. in jedem Mikrokanal, der den Verzweigungspunkt erster Ordnung 3.1 und die Eingänge 1.1 der Zellkultur-Kompartimente 1 miteinander verbindet, ist jeweils eine Einrichtung zur Volumenstromregelung 5 angeordnet. Jede der Einrichtungen zur Volumenstromregelung 5 weist dabei eine Verbindung mit einer zweiten Mikrokanalstruktur 7 auf. Jeweils jeder Ausgang 1.2 der Zellkultur-Kompartimente 1 ist über einen Mikrokanal mit einem Mündungspunkt 6.1 einer dritten Mikrokanalstruktur 6 verbunden, die in das Reservoir 2 mündet.

Im vorliegenden Beispiel sind die Einrichtungen zur Volumenstromregelung 5, die jeweils mit einem separaten Mikrokanal einer zweiten Mikrokanalstruktur 7 verbunden sind, jeweils mit einem Membranventil realisiert, das eine flexible Membran aufweist, die als Trennwand zwischen einem Mikrokanal der ersten Mikrokanalstruktur 3 und der Mikrokanalstruktur 7 ausgebildet ist. Über die zweite Mikrokanalstruktur 7 sind die Einrichtungen zur Volumenstromregelung 5 mit einem pneumatischen und/oder hydraulischen Druck beaufschlagbar, so dass eine von diesem Druck abhängige Wölbung der flexiblen Membran eine Querschnittsverengung des jeweiligen Mikrokanals der ersten Mikrokanalstruktur 3 verursacht, so dass der jeweils in die Zellkultur-Kompartimente 1 zuführbare Volumenstrom der aus dem Reservoir 2 geförderten Nährlösung verringert wird.

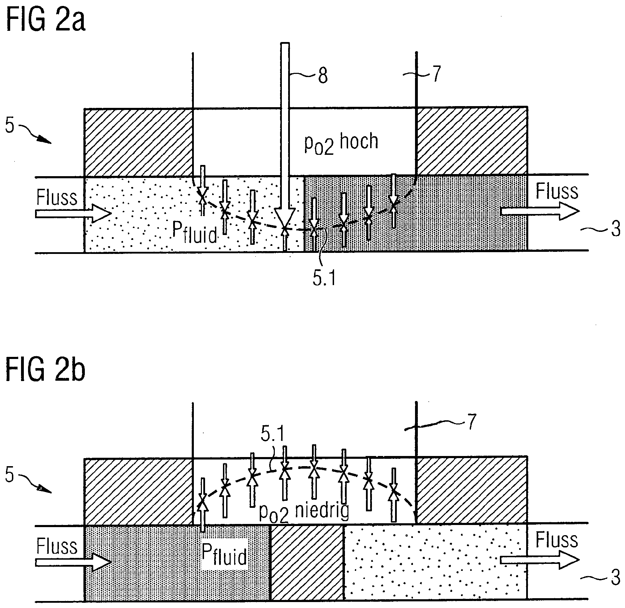

Die Figur 2a zeigt eine Schnittdarstellung einer mit einem Membranventil realisierten Einrichtung zur Volumenstromregelung 5. Dabei weist das Membranventil eine flexible Membran 5.1 auf. Das Bezugszeichen 3 kennzeichnet die erste Mikrokanalstruktur 3 bzw. einen Mikrokanal der ersten Mikrokanalstruktur 3, wobei die horizontalen Pfeile die Flussrichtung der Nährlösung anzeigen. Im vorliegenden Beispiel ist die flexible Membran 5.1 in Folge einer pneumatischen Druckbeaufschlagung auf die zweite Mikrokanalstruktur 7 in Richtung des ersten Mikrokanals gewölbt, so dass die von der Nährlösung durchströmbare Querschnittsfläche des ersten Mikrokanals 3 verengt ist. Bei der flexiblen Membran 5.1 handelt es sich im vorliegenden Beispiel um eine aus PDMS gebildete selektivpermeable Membran, die für Sauerstoff permeabel ist. Dadurch, dass die zweite Mikrokanalstruktur 7 im vorliegenden Beispiel gegenüber der ersten Mikrokanalstruktur 3 einen höheren Sauerstoffpartialdruck aufweist, kann Sauerstoff aus der zweiten Mikrokanalstruktur 7 in die ersten Mikrokanal 3 abgegeben werden, so dass die darin strömende Nährlösung mit Sauerstoff angereichert werden kann. Mit dem Bezugszeichen 8 ist der Laserstrahl eines Laser-Interferometers dargestellt, der auf die flexible Membran 5.1 gerichtet ist, so dass die Wölbung/Dehnung der flexiblen Membran 5.1 bestimmt werden kann. Der gleiche optische Zugang ermöglicht die Detektion des Sauerstoffpartialdrucks, beispielsweise durch Fluoreszenzlebensdauermessung an immobilisierten Partikeln.

Die Figur 2b zeigt ein weiteres Ausführungsbeispiel der Einrichtung zur Volumenstromregelung 5, die ebenfalls mit einem Membranventil realisiert ist, das eine flexible Membran 5.1 aufweist. Gegenüber der Figur 2a besteht der Unterschied darin, dass wie gezeigt, eine Veränderung des Querschnitts des ersten Mikrokanals 3 durch Aufbringen eines Unterdrucks (pneumatisch) an der zweiten Mikrokanalstruktur 7 realisiert ist. Während diese Ausführungsform der Einrichtung zur Volumenstromregelung 5 den Volumenstrom durch die erste Mikrokanalstruktur 3 bzw. durch einen Mikrokanal der ersten Mikrokanalstruktur 3 bei Anliegen eines Überdrucks bzw. Anliegen eines Normaldrucks, der gleich dem Druck in der ersten Mikrokanalstruktur 3 ist, sperrt, wird bei Anliegen eines Unterdrucks an der zweiten Mikrokanalstruktur 7 eine Wölbung in Richtung der zweiten Mikrokanalstruktur 7 erreicht und dadurch der Querschnitt der ersten Mikrokanalstruktur 3 erweitert.

In diesem Ausführungsbeispiel ist der Sauerstoffpartialdruck innerhalb der Mikrokanalstruktur 7 geringer gegenüber der ersten Mikrokanalstruktur 3, so dass bei Vorbeiströmen der Nährlösung an der Membran 5.1 Sauerstoff aus der Nährlösung entzogen wird.

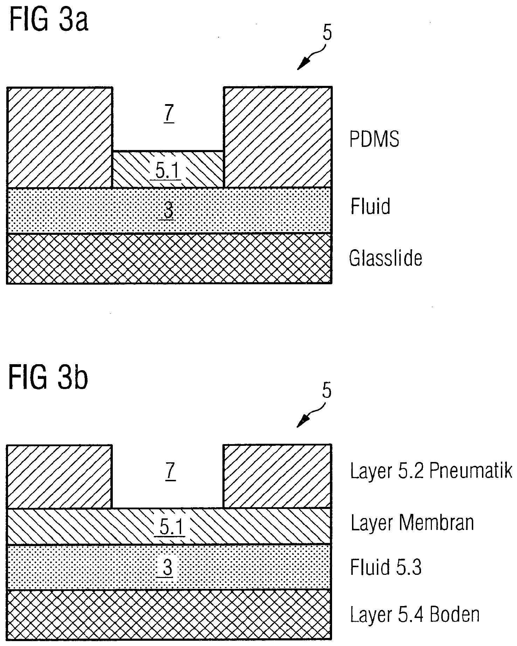

Die Figur 3a zeigt eine schematische Schnittdarstellung eines Ausführungsbeispiels der Einrichtung zur Volumenstromregelung 5, bei dem die flexible Membran 5.1 und die zweite Mikrokanalstruktur 7 aus einem PDMS Gussstück gebildet sind. Im vorliegenden Beispiel ist die erste Mikrokanalstruktur 3 bzw. ein Mikrokanal der Mikrokanalstruktur 3, in der/dem die Nährlösung strömt, zwischen einer Glasplatte (Glasslide) und dem Gussstück angeordnet ausgebildet.

Die Figur 3b zeigt eine weitere vorteilhafte Ausgestaltungsform der Einrichtung zur Volumenstromregelung 5 in einem Mehrschichtaufbau des erfindungsgemäßen Zirkulationssystems in Form einer schematischen Schnittdarstellung. Die einzelnen Schichten 5.1, 5.2, 5.3 und 5.4 sind dabei vorzugsweise aus PC gebildet - können aber auch aus PDMS, PP oder PET gebildet sein, wobei für jede Schicht auch unterschiedliche Polymerwerkstoffe eingesetzt werden können. Die einzelnen Schichten weisen entsprechend einer zu bildenden Struktur (Fördereinrichtung, Mikrokanalstruktur, Reservoir, Zellkultur-Kompartiment) miteinander korrespondierende Strukturen bzw. Aussparungen auf und sind dabei übereinander angeordnet miteinander laminiert. Im vorliegenden Beispiel ist die flexible Membran 5.1 durch eine PDMS Schicht repräsentiert, die eine Trennwand zwischen der in der ersten Schicht 5.2 ausgebildeten zweiten Mikrokanalstruktur 7 und der ersten Mikrokanalstruktur 3 bildet, die in der Schicht 5.3 ausgebildet ist. Das Bezugszeichen 5.4 kennzeichnet die untere Schicht, die die Mikrokanalstruktur 3 fluiddicht abschließt. Selbstverständlich kann oberhalb der Schicht 5.2 ebenfalls eine fluiddicht abschließende Schicht ausgebildet sein.

## Patentansprüche

1. Zirkulationssystem zur Vitalversorgung von Zellkulturen in einem mikrofluidischen Netzwerk, mit
mehreren Zellkultur-Kompartimenten (1), die jeweils mindestens einen Eingang (1.1) und mindestens einen Ausgang (1.2) aufweisen, und
mindestens einem Nährlösung enthaltenden Reservoir (2), das über eine erste Mikrokanalstruktur (3), die zumindest eine Verzweigung erster Ordnung (3.1) aufweist, mit mindestens einem Eingang jedes Zellkultur-Kompartiments (1) verbunden ist, und
mindestens einer Fördereinrichtung (4), mit der die Nährlösung aus dem Reservoir (2) über die Mikrokanalstruktur (3) in die Zellkultur-Kompartimente (1) förderbar ist,
in jedem Arm der Verzeigung erster Ordnung (3.1) und/oder im Verlauf jedes unmittelbar in einen Eingang (1.1) eines Zellkultur-Kompartiments (1) mündenden Mikrokanals der Mikrokanalstruktur (3), eine Einrichtung zur Volumenstromregelung (5) angeordnet ist, mit der ein Volumenstrom der Nährlösung regelbar ist und
die Einrichtung(en) zur Volumenstromregelung (5) mit einem Membranventil realisiert ist/sind, das eine flexiblen Membran (5.1) aufweist, wobei
die Einrichtung(en) zur Volumenstromregelung (5) einen konfokalen Sensor und/oder ein Laser-Interferometer als berührungslose sensorische Mittel (8) aufweist(en), mit denen die Wölbung/Dehnung der flexiblen Membran (5.1) sowie Drücke, Partialdrücke und/oder Volumenströme der Nährlösung und darin enthaltene oder zugeführte Fluide und/oder Konzentrationen von Fluiden und/oder Substanzen erfassbar sind.

2. Zirkulationssystem nach Anspruch 1 **dadurch gekennzeichnet, dass** die Einrichtung(en) zur Volumenstromregelung (5) mit einer zweiten Mikrokanalstruktur (7) verbunden ist/sind, über die mindestens ein Fluid und/oder mindestens eine Substanz in einen Mikrokanal der ersten Mikrokanalstruktur (3) in dosierter Form zuführbar ist/sind.

3. Zirkulationssystem nach Anspruch 1 oder 2, **dadurch gekennzeichnet, dass** die Einrichtung(en) zur Volumenstromregelung (5) mit einem MEMS-basierten Mikroventil realisiert ist/sind.

4. Zirkulationssystem nach einem vorhergehenden Anspruch, **dadurch gekennzeichnet, dass** die Einrichtung(en) zur Volumenstromregelung (5) mit einem magnetischen, elektromagnetischen, elektromotorischen, pneumatischen, elektropneumatischen, hydraulischen oder elektrohydraulischen Ventil realisiert ist/sind.

5. Zirkulationssystem nach dem vorhergehenden Anspruch, **dadurch gekennzeichnet, dass** die flexible Membran (5.1) als Trennwand zwischen der ersten Mikrokanalstruktur (3) und der zweiten Mikrokanalstruktur (7) ausgebildet ist und über die zweite Mikrokanalstruktur (7) mit einem aktivierbaren/deaktivierbaren vorgebbaren pneumatischen und/oder vorgebbaren hydraulischen Druck beaufschlagbar ist, wobei bei einer druckabhängigen Wölbung/Dehnung der flexiblen Membran (5.1), eine Änderung der von der Nährlösung durchströmbaren Querschnittsfläche eines Mikrokanals der ersten Mikrokanalstruktur (3) erreichbar ist.

6. Zirkulationssystem nach dem vorhergehenden Anspruch, **dadurch gekennzeichnet, dass** die flexible Membran (5.1) eine vorzugsweise aus Polydimethylsiloxan (PDMS), Polycarbonant (PC), Polypropylen (PP) oder Polyethylenterephthalat (PET) gebildete selektivpermeable Membran ist, die für mindestens ein Fluid, vorzugsweise ausgewählt aus der Gruppe aus Stickstoff, Sauerstoff, Kohlendioxid und/oder mindestens eine Substanz, vorzugsweise ausgewählt aus der Gruppe aus Stickstoff, Phosphat, Glucose, HSA, EGF permeabel ist, so dass ein Austausch des mindestens einen Fluids und/oder der mindestens einen Substanz zwischen einem Kanal der ersten Mikrokanalstruktur (3) und der dritten Mikrokanalanalstruktur (7) realisierbar ist.

7. Zirkulationssystem nach den vorhergehenden Ansprüchen 5 oder 6, **dadurch gekennzeichnet, dass** die flexible Membran(5.1) zumindest auf einer der Nährlösung zugewandten Seite eine amphiphile Oberflächenbeschichtung aufweist.

8. Zirkulationssystem nach den vorhergehenden Ansprüchen, **dadurch gekennzeichnet, dass** im Verlauf der ersten Mikrokanalstruktur (3) vor der mindestens einen Verzweigung erster Ordnung (3.1) und/oder in jedem Arm der Verzeigung erster Ordnung (3.1) und/oder im Verlauf jedes unmittelbar in einen Eingang (1.1) der Zellkultur-Kompartimente (1) mündenden Mikrokanal der ersten Mikrokanalstruktur (3), mindestens ein erster Oxygenator/Deoxygenator und/oder im Verlauf jeder aus einem Ausgang (1.2) eines Zellkultur-Kompartiments (1) in eine dritte Mikrokanalstruktur führende Mikrokanalverbindung vor einem Mündungspunkt (6.1) und/oder dahinter im Verlauf der dritten Mikrokanalstruktur (6) ein zweiter Oxygenator/Deoxygenator angeordnet ist/sind.

9. Zirkulationssystem nach einem vorhergehenden Anspruch, **dadurch gekennzeichnet, dass** das Zirkulationssystem im Mehrschichtaufbau realisiert ist, wobei mehrere Schichten miteinander laminierbar und flächig stoffschlüssig mit einander verbunden sind und in mindestens einer Schicht entsprechende Aussparungen für Mikrokanalstrukturen (3, 6, 7), für mindestens ein Reservoir (2), mindestens eine Fördereinrichtung (4), Zellkultur-Kompartimente (1) und Einrichtungen zur Volumenstromregelung (5) ausgebildet sind.

10. Verfahren zur Vitalversorgung von Zellkulturen in einem mikrofluidischen Netzwerk, bei dem
eine Nährlösung mit einer Fördereinrichtung (4) aus einem Reservoir (2) über mindestens eine Verzweigung erster Ordnung (3.1) einer sich verzweigenden Mikrokanalstruktur (3) eingangsseitig in mehrere Zellkultur-Kompartimente (1) gefördert wird, wobei
der Volumenstrom der Nährlösung in jedem Arm der Verzeigung erster Ordnung (3.1) und/oder im Verlauf jedes unmittelbar in einen Eingang (1.1) eines Zellkultur-Kompartiments (1) mündenden Mikrokanals der Mikrokanalstruktur (3) separat geregelt wird und
die Volumenstromregelung mit einem Membranventil realisiert wird, das eine flexiblen Membran (5.1) aufweist, wobei
die Wölbung/Dehnung der flexiblen Membran (5.1) sowie Drücke, Partialdrücke und/oder Volumenströme der Nährlösung und darin enthaltene oder zugeführte Fluide und/oder Konzentrationen von Fluiden und/oder Substanzen berührungslos mit einem konfokalen Sensor und/oder einem Laser-Interferometer als sensorische Mittel (8) berührungslos erfasst werden.

11. Verfahren nach Anspruch 10, **dadurch gekennzeichnet, dass** zur Volumenstromregelung der Nährlösung eine Einrichtung zur Volumenstromregelung (5) eingesetzt wird, die ein Membranventil mit einer flexiblen Membran (5.1) aufweist, wobei die flexible Membran (5.1) als Trennwand zwischen der ersten Mikrokanalstruktur (3) und einer zweiten Mikrokanalstruktur (7) ausgebildet ist, wobei über die zweite Mikrokanalstruktur (7) ein aktivierbarer/deaktivierbarer vorgebbarer pneumatischer und/oder vorgebbarer hydraulischer Druck beaufschlagt wird, dabei die flexible Membran (5.1) derart gewölbt/gedehnt wird, dass die von der Nährlösung durchströmbare Querschnittsfläche eines Mikrokanals der ersten Mikrokanalstruktur (3) verändert wird.

12. Verfahren nach Anspruch 10 oder 11, **dadurch gekennzeichnet, dass** als flexible Membran eine vorzugsweise aus PDMS, PC, PP oder PET gebildete selektivpermeable Membran verwendet wird, über die zwischen einem Mikrokanal der ersten Mikrokanalstruktur (3) und der dritten Mikrokanalanalstruktur (7) mindestens ein Fluid, vorzugsweise ausgewählt aus der Gruppe aus Stickstoff, Sauerstoff, Kohlendioxid und/oder mindestens eine Substanz, vorzugsweise ausgewählt aus der Gruppe aus Stickstoff, Phosphat, Glucose, HSA, EGF ausgetauscht und/oder dosiert wird.

## Claims

1. Circulation system for vital supply of cell cultures in a microfluidic network, comprising:
multiple cell culture compartments (1), each having at least one inlet (1.1) and at least one outlet (1.2), and
at least one reservoir (2) containing nutrient solution, which is connected to at least one inlet of each cell culture compartment (1) via a first microchannel structure (3) which has at least one first-order branch (3.1), and
at least one conveying device (4) with which the nutrient solution can be conveyed from the reservoir (2) via the microchannel structure (3) into the cell culture compartments (1),
a volumetric flow regulator (5), with which a volumetric flow of the nutrient solution can be regulated, is arranged in each arm of the first-order branch (3.1) and/or in the course of each microchannel, which opens into an inlet (1.1) of a cell culture compartment (1), of the microchannel structure (3), and
the volumetric flow regulator(s) (5) is/are embodied with a membrane valve which has a flexible membrane (5.1), wherein
the volumetric flow regulator(s) (5) feature(s) a confocal sensor and/or a laser interferometer as contactless sensing means (8) which make it possible to sense curvature/extension of the flexible membrane (5.1) as well as pressures, partial pressures and/or volumetric flows of the nutrient solution and fluids contained therein or supplied thereto and/or concentrations of fluids and/or substances.

2. Circulation system according to claim 1, **characterized in that** the volumetric flow regulator(s) (5) is/are connected to a second microchannel structure (7) via which at least one fluid and/or at least one substance can be supplied into a microchannel of the first microchannel structure (3) in dosed quantities.

3. Circulation system according to claim 1 or 2, **characterized in that** the volumetric flow regulators (s) (5) are embodied with a MEMS-based microvalve.

4. Circulation system according to one of the preceding claims, **characterized in that** the volumetric flow regulator(s) (5) is/are embodied with a magnetic, electromagnetic, electromotive, pneumatic, electropneumatic, hydraulic or electrohydraulic valve.

5. Circulation system according to one of the preceding claims, **characterized in that** the flexible membrane (5.1) is designed as a partition wall between the first microchannel structure (3) and the second microchannel structure (7) and can be subjected to an activatable/deactivatable predeterminable pneumatic pressure and/or a predeterminable hydraulic pressure, wherein in the case of a pressure-dependent curvature/extension of the flexible membrane (5.1), change of the cross-sectional surface, which can be passed through by the nutrient solution, of a microchannel of the first channel structure (3) can be attained.

6. Circulation system according to one of the preceding claims, **characterized in that** the flexible membrane (5.1) is a selectively permeable membrane, preferably formed from polydimethylsiloxane (PDMS), polycarbonate (PC), polypropylene (PP) or polyethyleneterephtalate (PET), which is permeable to at least one fluid, preferably selected from the group consisting of nitrogen, oxygen, carbon dioxide and/or at least one substance, preferably selected from the group consisting of nitrogen, phosphate, glucose, HSA, EGF, so that exchange of the at least one fluid and/or the at least one substance can be realized between a channel of the first microchannel structure (3) and the third microchannel structure (7).

7. Circulation system according to one of the preceding claims 5 or 6, **characterized in that** the flexible membrane (5.1) features an amphiphilic surface coating at least on a side facing the nutrient solution.

8. Circulation system according to the preceding claims, **characterized in that** at least one first oxygenator/deoxygenator is/are arranged in the course of the first microchannel structure (3) downstream of the at least one first-order branch (3.1) and/or in each arm of the first-order branch (3.1) and/or in the course of each microchannel, which opens directly into an inlet (1.1) of the cell culture compartments (1), of the first microchannel structure (3), and/or a second oxygenator/deoxygenator is/are arranged in the course of each microchannel connection leading from an outlet (1.2) of a cell culture compartment (1) into a third microchannel structure downstream of a junction point (6.1) and/or upstream thereof in the course of the third microchannel structure (6).

9. Circulation system according to one of the preceding claims, **characterized in that** the circulation system is realized with a multi-layer structure, wherein several layers can be laminated with each other and are areally and integrally joined to one another, and corresponding recesses for microchannel structures (3, 6, 7), for at least one reservoir (2), at least one conveying device (4), cell culture compartments (1) and volumetric flow regulators (5) are formed in at least one layer.

10. Method for vital supply of cell cultures in a microfluidic network, in which
nutrient solution is conveyed from a reservoir (2) using a conveying device (4) via at least one first-order branch (3.1) of a branched microchannel structure (3) at an inlet side into multiple cell culture compartments (1), wherein
the volumetric flow of the nutrient solution is separately regulated in each arm of the first-order branch (3.1) and/or in the course of each microchannel, which opens directly into an inlet (1.1) of a cell culture compartment (1), of the microchannel structure (3), and the volumetric flow regulation is realised with the aid of a membrane valve which has a flexible membrane (5.2), wherein
curvature/extension of the flexible membrane (5.1) as well as pressures, partial pressures and/or volumetric flows of the nutrient solution and fluids contained therein or supplied thereto and/or concentrations of fluids and/or substances are sensed in a contactless manner using a confocal sensor and/or a laser interferometer as sensing means (8).

11. Method according to claim 10, **characterized in that** a volumetric flow regulator (5) is employed for volumetric flow regulation of the nutrient solution and has a membrane valve with a flexible membrane (5.1), wherein the flexible membrane (5.1) is designed as a partition wall between the first microchannel structure (3) and a second microchannel structure (7), wherein an activatable/deactivatable, predeterminable pneumatic and/or a predeterminable hydraulic pressure is applied via the second microchannel structure (7), resulting **in that** the flexible membrane (5.2) is curved/extended in such a manner that the cross-sectional surface, which can be passed through by the nutrient solution, of a microchannel of the first microchannel structure (3) is changed.

12. Method according to claim 10 or 11, **characterized in that** a selectively permeable membrane, preferably formed from PDMS, PC, PP or PET, is used as the flexible membrane, via which at least one fluid, preferably selected from the group consisting of nitrogen, oxygen, carbon dioxide and/or at least one substance, preferable selected from the group consisting of nitrogen, phosphate, glucose, HSA, EGF, is exchanged between a microchannel of the first microchannel structure (3) and the third microchannel structure (3) or is supplied in dosed quantities.

## Revendications

1. Système de circulation pour l'alimentation vitale de cultures cellulaires dans un réseau micro-fluidique, avec
plusieurs compartiments de cultures cellulaires (1), comprenant chacun au moins une entrée (1.1) et au moins une sortie (1.2) et
au moins un réservoir (2) contenant une solution nutritive, relié, par l'intermédiaire d'une première structure à micro-canaux (3), qui comprend au moins un embranchement de premier ordre (3.1), avec au moins une entrée de chaque compartiment de cultures cellulaires (1) et
au moins un dispositif de transport (4) avec lequel la solution nutritive peut être transportée du réservoir (2) vers les compartiments de cultures cellulaires (1) par l'intermédiaire de la structure à micro-canaux (3),
dans chaque branche de l'embranchement de premier ordre (3.1) et/ou sur le trajet de chaque micro-canal de la structure à micro-canaux (3), débouchant directement dans une entrée (1.1) d'un compartiment de culture cellulaire (1), se trouve un dispositif de régulation du débit volumique (5) avec lequel un débit volumique de la solution nutritive peut être régulé et
le(s) dispositif(s) de régulation du débit volumique (5) est/sont réalisé(s) avec une vanne à membrane qui comprend une membrane flexible (5.1), moyennant quoi
le(s) dispositif(s) de régulation du débit volumique (5) comprend/comprennent un capteur confocal et/ou un interféromètre laser en tant que moyens de capteurs sans contact (8), avec lesquels la courbure/dilatation de la membrane flexible (5.1) ainsi que les pressions, les pressions partielles et/ou les débits volumiques de la solution nutritive et des fluides qui y sont contenus ou introduits et/ou les concentrations de fluides et/ou de substances peuvent être mesurés.

2. Système de circulation selon la revendication 1, **caractérisé en ce que** le(s) dispositif(s) de régulation du débit volumique (5) est/sont relié(s) à une deuxième structure à micro-canaux (7) par l'intermédiaire de laquelle au moins un fluide et/ou au moins une substance peut/peuvent être introduit(s) dans un micro-canal de la première structure à micro-canaux (3) sous une forme dosée.

3. Système de circulation selon la revendication 1 ou 2, **caractérisé en ce que** le(s) dispositif(s) de régulation du débit volumique (5) est/sont réalisé(s) avec une micro-vanne basée sur MEMS.

4. Système de circulation selon une revendication précédente, **caractérisé en ce que** le(s) dispositif(s) de régulation du débit volumique (5) est/sont réalisé(s) avec une vanne magnétique, électromagnétique, électromotrice pneumatique, électropneumatique, hydraulique ou électrohydraulique.

5. Système de circulation selon la revendication précédente, **caractérisé en ce que** la membrane flexible (5.1) est conçue comme une cloison entre la première structure à micro-canaux (3) et la deuxième structure à micro-canaux (7) et peut être alimentée, par l'intermédiaire de la deuxième structure à micro-canaux (7), avec une pression pneumatique activable/désactivable prédéterminée et/ou une pression hydraulique activable/désactivable prédéterminée, moyennant quoi, il est possible d'obtenir lors d'une courbure/dilatation de la membrane flexible (5.1), une variation de la surface de section d'un micro-canal pouvant être traversée par la solution nutritive de la première structure à micro-canaux (3).

6. Système de circulation selon la revendication précédente, **caractérisé en ce que** la membrane flexible (5.1) est de préférence une membrane sélectivement perméable constituée de polydiméthylsiloxane (PDMS), de polycarbonate (PC), de polypropylène (PP) ou de polyéthylènetéréphthalate (PET), perméable pour au moins un fluide sélectionné de préférence parmi le groupe constitué de l'azote, de l'oxygène, du dioxyde de carbone et/ou d'au moins une substance sélectionnée de préférence dans le groupe constitué de l'azote, d'un phosphate, du glucose, de HSA, de EGF, de façon à ce qu'un échange de l'au moins un fluide et/ou de l'au moins une substance entre un canal de la première structure à micro-canaux (3) et de la troisième structure à micro-canaux (7) puisse être réalisé.

7. Système de circulation selon les revendications précédentes 5 ou 6, **caractérisé en ce que** la membrane flexible (5.1) comprend au moins un revêtement de surface amphiphile sur un côté orienté vers la solution nutritive.

8. Système de circulation selon les revendications précédentes, **caractérisé en ce que**, sur le trajet de la première structure à micro-canaux (3), avant l'au moins un embranchement de premier ordre (3.1) et/ou dans chaque branche de l'embranchement de premier ordre (3.1) et/ou sur le trajet de chaque micro-canal de la première structure à micro-canaux (3), débouchant directement dans une entrée (1.1) des compartiments de cultures cellulaires (1), se trouve au moins un premier oxygénateur/désoxygénateur et/ou sur le trajet de chaque liaison à micro-canal conduisant, à partir d'une sortie (1.2) d'un compartiment de cultures cellulaires (1), vers une troisième structure à micro-canaux, avant un point d'embouchure (6.1) et/ou derrière celui-ci, sur le trajet de la troisième structure à micro-canaux (6), se trouve un deuxième oxygénateur/désoxygénateur.

9. Système de circulation selon une revendication précédente, **caractérisé en ce que** le système de circulation est réalisée avec une structure multi-couches, plusieurs couches pouvant être laminées entre elles et étant reliées entre elles sur toute leur surface par liaison de matière et, dans au moins une couche sont réalisés des évidements correspondants pour des structures à micro-canaux (3, 6, 7), pour au moins un réservoir (2), au moins un dispositif de transport (4), des compartiments de cultures cellulaires (1) et des dispositifs de régulation du débit volumique (5).

10. Procédé d'alimentation vitale de cultures cellulaires dans un réseau micro-fluidique, dans lequel
une solution nutritive est transportée, avec un dispositif de transport (4), d'un réservoir (2), par l'intermédiaire d'au moins un embranchement de premier ordre (3.1) d'une structure à micro-canaux (3) à ramifications, côté entrée vers plusieurs compartiments cellulaires (1), moyennant quoi,
le débit volumique de la solution nutritive est régulé séparément dans chaque branche de l'embranchement de premier ordre (3.1) et/ou sur le trajet de chaque micro-canal de la structure à micro-canaux (3) débouchant directement dans une entrée (1.1) d'un compartiment de cultures cellulaires (1) et la régulation du débit volumique est réalisée avec une vanne à membrane qui comprend une membrane flexible (5.1), moyennant quoi,
la courbure/dilatation de la membrane flexible (5.1) ainsi que les pressions, pressions partielles et/ou débits volumiques de la solution nutritive et des fluides qui y sont contenus ou introduits et/ou les concentrations de fluides et/ou de substances sont mesurés sans contact par un capteur confocal et/ou un interféromètre laser en qualité de moyens de capteurs (8).

11. Procédé selon la revendication 10, **caractérisé en ce que**, pour la régulation du débit volumique de la solution nutritive, un dispositif de régulation de débit volumique (5) est utilisé, qui comprend une vanne à membrane avec une membrane flexible (5.1), la membrane flexible (5.1) étant conçue comme une cloison entre la première structure à micro-canaux (3) et une deuxième structure à micro-canaux (7), moyennant quoi, par l'intermédiaire de la deuxième structure à micro-canaux (7), une pression pneumatique activable/désactivable prédéterminée et/ou une pression hydraulique activable/désactivable prédéterminée est appliquée, la membrane flexible (5.1) est alors courbée/dilatée de façon à ce que la surface de section d'un micro-canal de la première structure à micro-canaux (3) traversée par la solution nutritive soit modifiée.

12. Procédé selon la revendication 10 ou 11, **caractérisé en ce que**, en tant que membrane flexible, une membrane sélectivement perméable constituée de préférence de PDMS, de PC, de PP ou de PET est utilisée, par l'intermédiaire de laquelle, entre un micro-canal de la première structure à micro-canaux (3) et la troisième structure à micro-canaux (7), au moins un fluide, sélectionné de préférence dans le groupe constitué de l'azote, de l'oxygène, du dioxyde de carbone et/ou au moins une substance, sélectionnée de préférence dans le groupe constitué de l'azote, d'un phosphate, du glucose, de HSA, de EGF est échangé et/ou dosé.
